# EUROPÄISCHE PATENTANMELDUNG

(11) **EP 1 801 718 A1**
(43) Veröffentlichungstag der Anmeldung: **27.06.2007**
(21) Anmeldenummer: 05028021.3
(22) Anmeldetag: 21.12.2005
(51) Int. Cl.: G06F 19/00, A61M 5/142, A61M 5/172

(54) **Verfahren zum Betrieb einer computergesteuerten Dosiervorrichtung für flüssige Medikamente bei reisebedingter Zeitverschiebung**

(71) Anmelder: F. HOFFMANN-LA ROCHE AG, 4070 Basel (CH); Roche Diagnostics GmbH, 68305 Mannheim (DE)
(72) Erfinder: Remde, Axel, 3432 Lützelflüh-Goldbach (CH)
(74) Vertreter: Blum, Rudolf Emil

(57) **Zusammenfassung**

Die Erfindung betrifft eine computergestützte Insulinpumpe, mit welcher automatisch eine stufenweise Anpassung des basalen Förderprofils bei einer reisebedingten Zeitverschiebung vorgenommen werden kann, indem der Pumpensteuerung die Ortszeit am Zielort mitgeteilt wird und diese hieraus gemäss einem abgespeicherten Algorithmus ein oder mehrere Übergangs-Förderprofile errechnet, die sodann vorübergehend zur automatischen Steuerung der Insulinpumpe eingesetzt werden, bis die Zeitumstellung vollständig vollzogen ist.

Hierdurch wird es möglich, auf die gemäss dem Stand der Technik bei grösseren Zeitverschiebungen erforderliche manuelle, schrittweise Umstellung zu verzichten, was den Betrieb von Dosiervorrichtungen für flüssige Medikamente, insbesondere von Insulinpumpen, bei Reisen über mehrere Zeitzonengrenzen einfacher, komfortabler und sicherer macht.

## Beschreibung

Die vorliegende Erfindung betrifft ein Verfahren zum Betrieb einer Dosiervorrichtung für flüssige Medikamente mit einer computergestützten Steuerung bei reisebedingter Verschiebung der Ortszeit, die Anwendung des Verfahrens zum Betrieb einer computergesteuerten Insulinpumpe, eine Dosiervorrichtung, insbesondere zur Durchführung des Verfahrens, sowie die Verwendung der Dosiervorrichtung zur dosierten Förderung von Insulin gemäss den Oberbegriffen der unabhängigen Patentansprüche.

Die automatisierte Zuführung von flüssigen Medikamenten in den Körper eines Patienten kommt bevorzugterweise dann zur Anwendung, wenn ein permanenter und über den Tag gesehen variierender Bedarf an einem subkutan zu verabreichenden Medikament vorliegt. So kommen z.B. zur Behandlung von Diabetes melitus computergesteuerte Insulinpumpen zum Einsatz, welche eine Insulinversorgung des Patienten ermöglichen, die den physiologischen Gegebenheiten eines Nicht-Diabetikers in guter Näherung entspricht. Die sogenannte basale Grundversorgung des Körpers mit Insulin geschieht hierbei quasi-kontinuierlich durch die häufige Abgabe kleinster Insulinmengen in den Blutkreislauf des Patienten. Die Insulinabgabe erfolgt dabei nicht mit einer konstanten Förderrate, sondern mit über den Tag wechselnden Förderraten gemäss einem Abgabe-Tagesprofil, welches vom Arzt individuell für den Patienten festgelegt und in der Pumpesteuerung mit Bezug auf die pumpeninterne Uhr, welche die Steuerungsfunktionen verwaltet, abgespeichert wird.

Beim reisebedingten Überschreiten von Zeitzonengrenzen ist es deshalb erforderlich, dieses auch als basales Förderprofil bezeichnete Abgabe-Tagesprofil an die Zeitverschiebung zwischen Ausgangs- und Zielort der Reise anzupassen, da sich in der Regel der Tagesrhythmus mit der Ortszeit verschiebt, und mit diesem das Insulinbedarfsprofil des Reisenden. Um dieser Verschiebung Rechnung zu tragen, kann der Pumpenträger nun beispielsweise die pumpeninterne Uhr, z.B. bei Beginn oder Ende der Reise, auf die Ortszeit am Zielort einstellen, was bei geringen Zeitverschiebungen von bis zu zwei Stunden meist ohne Probleme möglich ist. Bei einer Reise jedoch, bei der innerhalb von einer kurzen Reisezeit eine grosse Zeitverschiebung entsteht, z.B. bei einer Flugreise über mehrer Zeitzonengrenzen hinweg, ist dieses Vorgehen nicht vorteilhaft, da sich der physiologische Rhythmus des Körpers einer derartigen Zeitverschiebung nicht schlagartig, sondern nur langsam, gegebenenfalls über etliche Tage hinweg, anpasst. Bei einer schlagartigen Umstellung würde daher anfänglich eine erhebliche Diskrepanz zwischen dem physiologischem Insulinbedarfsprofil, welches zunächst noch weitgehend am vorherigen Tagesrythmus am Heimatort orientiert ist, und dem realen Insulin-Abgabeprofil der Insulinpumpe entstehen.

Es wird daher von den meisten Ärzten empfohlen, die Umstellung des Abgabe-Tagesprofils der Insulinpumpe bei grösseren Zeitverschiebungen in mehreren Stufen und über einen längeren Zeitraum vorzunehmen. Dies erfolgt beispielsweise derart, dass das basale Abgabe-Tagesprofil bei Ankunft am Zielort oder alternativ bei Reiseantritt gegenüber der "Heimatzeit" um zwei Stunden verschoben wird und anschliessend an jedem zweiten Tag eine weitere Verschiebung um zwei Stunden vorgenommen wird, bis die vollständige Anpassung an die Ortszeit des Zielortes erreicht ist. Dabei gibt es grundsätzlich zwei verschiedene Vorgehensweisen:
a) Zu den entsprechenden Zeitpunkten stellt der Pumpenträger die pumpeninterne Uhr jeweils um zwei Stunden vor bzw. zurück. Ist die Zeitverschiebung kein ganzzahliges Vielfaches von zwei Stunden, erfolgt im letzten Schritt eine Verschiebung um den Restbetrag.
b) Die Pumpenuhr wird bei Ankunft sofort auf die neue Ortszeit umgestellt. Die stufenweise Verschiebung erfolgt dadurch, dass zu den gewählten Umstellungszeitpunkten jeweils das gesamte Abgabe-Tagesprofil neu programmiert wird.

Auch wenn diese beiden Verfahren die gewünschte schrittweise Umstellung des Förderprofils ermöglichen, so sind sie doch mit erheblichen Nachteilen verbunden.

Die schrittweise Umstellung der Pumpenuhr ist zwar relativ einfach, bedeutet jedoch, dass die angezeigte Zeit während der Umstellungsphase nicht der Ortszeit des Zielortes entspricht. Dies ist insofern problematisch, als die Pumpenuhr von vielen Pumpenträgern wie eine Armbanduhr verwendet wird, was dann während der Umstellungsphase nicht möglich ist und deshalb zu Unregelmässigkeiten des Tagesablaufs führen kann. Ferner ist zu bedenken, dass die sogenannte "Pumpenhistory", d.h. die pumpeninterne Protokollierung aller relevanten Vorgänge, wie z.B. die Abgabe von Bolus-Insulin bei Mahlzeiten, mit Referenz zur Pumpenuhr erfolgt, wodurch die Auswertung der History zur Therapieprotokollierung während der Umstellungsphase erschwert wird. So können beispielsweise Mahlzeitenboli mitten in der Nacht in der Pumpenhistory erscheinen.

Die häufige Umprogrammierung des basalen Förderprofils entsprechend der zweiten üblichen Methode ist hingegen aufwändig und fehlerträchtig und schon deshalb wenig praktikabel.

Bei beiden Varianten besteht zudem die Gefahr, dass Umstellungen vergessen werden.

Es stellt sich daher die Aufgabe, Verfahren und Vorrichtungen zur Verfügung zu stellen, welche die Nachteile des Standes der Technik nicht aufweisen oder diese zumindest teilweise vermeiden.

Diese Aufgabe wird durch das Verfahren und die Dosiervorrichtung gemäss den unabhängigen Patentansprüchen gelöst.

Demgemäss umfasst ein erster Aspekt der Erfindung ein Verfahren zum Betrieb einer computergesteuerten Dosiervorrichtung für flüssige Medikamente, z.B. eine Insulinpumpe, bei reisebedingter Verschiebung der Ortszeit. Dabei wird die Dosiervorrichtung zuerst an einem ersten Ort, normalerweise der Ausgangsort der Reise bzw. der Heimatort, an welchem eine erste Ortszeit, die Heimatzeit, gilt, mit der computergestützten Steuerung derartig gesteuert, dass sie das flüssige Medikament automatisch gemäss einem intern gespeicherten und sich täglich wiederholenden basalen Grundbedarfsprofil, dem anspruchsgemässen ersten Abgabe-Tagesprofil, abgibt. Ausgehend von dem ersten Ort wird die Dosiervorrichtung an einen zweiten Ort gebracht, normalerweise der Zielort der Reise, an welchem eine zweite Ortszeit gilt, welche von der ersten Ortszeit um die anspruchsgemässe erste Zeitverschiebung abweicht. Dabei wird die Dosiervorrichtung anschliessend an die Abgabe von flüssigem Medikament gemäss dem ersten Abgabe-Tagesprofil vorübergehend zur Anpassung an die erste Zeitverschiebung mit der Steuerung automatisch derart gesteuert, dass sie das flüssige Medikament gemäss einem oder mehreren, vom ersten Abgabe-Tagesprofil und dem um die erste Zeitverschiebung verschobenen ersten Abgabe-Tagesprofil verschiedenen, weiteren Abgabe-Tagesprofilen abgibt, also mit anderen Worten gemäss einem Abgabeprofil, welches weder dem Abgabe-Tagesprofil vor Einleitung der Umstellungsphase auf die zweite Ortszeit entspricht noch dem Abgabe-Tagesprofil nach vollendeter Umstellung des Betriebs auf die zweite Ortszeit. Dabei wird das oder werden die weiteren Abgabe-Tagesprofile mit der Steuerung in Abhängigkeit der ersten Zeitverschiebung ermittelt.

In einer bevorzugten Ausführungsform des erfindungsgemässen Verfahrens wird die Dosiervorrichtung nach dem zweiten Ort an einen weiteren Ort, den anspruchsgemässen dritten Ort mit einer dritten, von der zweiten Ortszeit um die anspruchsgemässe zweite Zeitverschiebung verschiedenen Ortszeit gebracht, was beispielsweise dann der Fall ist, wenn die Reise fortgesetzt wird an einen weiteren Ort mit anderer Ortszeit oder wenn die Rückreise an den Ausgangsort, also an den anspruchsgemässen ersten Ort, angetreten wird. Dabei wird die Dosiervorrichtung vorübergehend zur Anpassung an die zweite Zeitverschiebung mit der Steuerung derartig gesteuert, dass sie das flüssige Medikament automatisch gemäss einem oder mehreren weiteren Abgabe-Tagesprofilen abgibt, welches oder welche von dem zuletzt am zweiten Ort verwendeten Tages-Abgabeprofil und dem um die vorzeichenrichtige Summe aus erster und zweiter Zeitverschiebung verschobenen ersten Abgabe-Tagesprofil verschieden ist oder sind. Diese weiteren Abgabe-Tagesprofile werden mit der Steuerung gemäss einem Algorithmus in Abhängigkeit von der zweiten Zeitverschiebung ermittelt. Bei einer Rückreise an den ersten Ort entspricht die zweite Zeitverschiebung der ersten Zeitverschiebung mit umgekehrtem Vorzeichen.

Dabei ist es für den Fall, dass das zuletzt am zweiten Ort verwendete weitere Abgabe-Tagesprofil von einem Abgabe-Tagesprofil, welches dem um die erste Zeitverschiebung verschobenen ersten Abgabe-Tagesprofil entspricht, abweicht, also die Umstellung auf die zweite Ortszeit zum Zeitpunkt der Weiterreise noch nicht vollständig vollzogen ist, von Vorteil, wenn diese Abweichung bei der Ermittlung der weiteren, in Abhängigkeit von der zweiten Zeitverschiebung ermittelten weiteren Abgabe-Tagesprofile berücksichtigt wird, so dass nur die effektiv zur Anpassung der aktuellen Abgabe-Situation an die dritte Ortszeit erforderlichen Anpassungsschritte vorgenommen werden.

Ein zweiter Aspekt der Erfindung betrifft eine Dosiervorrichtung für flüssige Medikamente, welche bevorzugterweise zur Durchführung des Verfahrens gemäss dem ersten Aspekt der Erfindung geeignet ist. Die Dosiervorrichtung umfasst eine Fördereinrichtung zur Förderung des flüssigen Medikaments von einem Medikamentenreservoir zu einer Abgabeleitung, z.B. einer Infusionskanüle, und eine computergestützte Steuerung zur automatischen Steuerung der Fördereinrichtung derart, dass diese das flüssige Medikament automatisch gemäss einem in der Steuerung abgespeicherten und sich täglich wiederholenden basalen Grundbedarfsprofil, dem anspruchsgemässen ersten Abgabe-Tagesprofil, fördert. Die Steuerung ist zudem derartig ausgestaltet, dass mit ihr bei einer anspruchsgemässen ersten Zeitverschiebung ein oder mehrere vom ersten Abgabe-Tagesprofil und dem um die erste Zeitverschiebung verschobenen ersten Abgabe-Tagesprofil verschiedene weitere Abgabe-Tagesprofile gemäss einem steuerungsinternen Algorithmus in Abhängigkeit von der ersten Zeitverschiebung ermittelbar und zwecks Anpassung an die erste Zeitverschiebung vorübergehend zur automatischen Steuerung der Fördereinrichtung verwendbar sind. Die erste Zeitverschiebung kann sich beispielsweise durch eine Flugreise von einem ersten Ort mit einer ersten Ortszeit zu einem zweiten Ort mit einer anderen, zweiten Ortszeit ergeben, oder kann auch durch eine Zeitumstellung von Winterzeit auf Sommerzeit oder durch einen Wechsel des Tagesrythmus eines Patienten z.B. bei Umstellung von Tagarbeit auf Nachtarbeit, hervorgerufen sein.

In einer bevorzugten Ausführungsform der erfindungsgemässen Dosiervorrichtung ist die Steuerung derartig ausgestaltet, dass mit ihr bei einer an die erste Zeitverschiebung anschliessenden zweiten Zeitverschiebung mindestens ein weiteres Abgabe-Tagesprofil gemäss einem steuerungsinternen Algorithmus in Abhängigkeit von der zweiten Zeitverschiebung ermittelbar und vorübergehend zur automatischen Steuerung der Dosiervorrichtung verwendbar ist, welches von dem direkt zuvor verwendeten Abgabe-Tagesprofil und dem um die vorzeichenrichtige Summe aus der ersten und der zweiten Zeitverschiebung verschobenen ersten Abgabe-Tagesprofil verschieden ist. Die zweite Zeitverschiebung kann beispielsweise durch Weiterreise an einen dritten Ort mit einer anderen Ortszeit als die Ortszeit am zweiten Ort oder durch Rückreise an den ersten Ort, d.h. Rückgängigmachung der ersten Zeitverschiebung, hervorgerufen sein.

Dabei ist es bevorzugt, wenn die Steuerung der Dosiervorrichtung zudem derartig ausgestaltet ist, dass mit ihr für den Fall, dass das zuletzt zur Anpassung an die erste Zeitverschiebung verwendete Tages-Abgabeprofil von einem um die erste Zeitverschiebung verschobenen ersten Abgabe-Tagesprofil abweicht, was typischerweise dann den Fall ist, wenn eine mehrstufige Anpassung an die erste Zeitverschiebung noch nicht abgeschlossen ist, diese Abweichung bei der Ermittlung der in Abhängigkeit von der zweiten Zeitverschiebung ermittelten weiteren Abgabe-Tagesprofile optional oder automatisch mitberücksichtigt werden kann.

Durch das erfindungsgemässe Betriebsverfahren und die erfindungsgemässe Dosiervorrichtung wird es erstmals möglich, auf die gemäss dem Stand der Technik bei grösseren Zeitverschiebungen erforderliche manuelle, schrittweise Umstellung zu verzichten, was den Betrieb von Dosiervorrichtungen für flüssige Medikamente, insbesondere von Insulinpumpen, bei Reisen über mehrere Zeitzonengrenzen hinweg einfacher, komfortabler und sicherer macht und zudem auch feinere Umstellungsschritte ermöglicht, welche während der Übergangsphase eine bessere Anpassung des Abgabe-Tagesprofils an das physiologische Bedarfsprofil des Patienten zulassen.

In einer bevorzugten Ausführungsform des erfindungsgemässen Verfahrens und der erfindungsgemässen Anordnung (im Folgenden summarisch als "die Erfindung" bezeichnet), entspricht ein Teil oder entsprechen alle der weiteren Abgabe-Tagesprofile einem zeitlich um eine bestimmte Zeitverschiebung verschobenen ersten Abgabe-Tagesprofil. Hierdurch ergibt sich der Vorteil, dass die Umstellungseingriffe der Steuerung überschaubar und nachvollziehbar bleiben, was die Überprüfung der korrekten Funktionsweise der Dosiervorrichtung erleichtert.

Dabei ist es für den Fall, dass eine etwaige noch nicht vollständig vollzogene Anpassung an die erste Zeitverschiebung bei der Ermittlung weiterer Abgabe-Tagesprofile in Abhängigkeit von einer auf die erste Zeitverschiebung folgenden zweiten Zeitverschiebung berücksichtigt wird, von Vorteil, wenn dies in der Weise geschieht, dass der Differenzbetrag zwischen der ersten Zeitverschiebung und dem beim letzten zur Anpassung an diese verwendeten Abgabe-Tagesprofil bereits realisierten Anteil der ersten Zeitverschiebung vorzeichenrichtig mit der zweiten Zeitverschiebung aufsummiert wird, diese also um diesen Differenzbetrag vergrössert oder verkleinert wird, und anschliessend die weiteren Abgabe-Tagesprofile in Abhängigkeit von dieser korrigierten zweiten Zeitverschiebung ermittelt werden. Auf diese Weise kann auch eine Verkettung von noch nicht abgeschlossenen Anpassungen an Zeitverschiebungen einfach und überschaubar vorgenommen werden, ohne dass es dabei zu starken Abweichungen der vorübergehend zur Steuerung verwendeten weiteren Abgabe-Tagesprofile vom jeweiligen physiologischen Bedarfsprofil kommt.

Zudem ist es für den Fall, dass die weiteren Abgabe-Tagesprofile einem zeitlich um eine bestimmte Zeitverschiebung verschobenen ersten Abgabe-Tagesprofilen entsprechen, von Vorteil, wenn die Zeitverschiebungsdifferenz zwischen jeweils direkt aufeinander folgenden Abgabe-Tagesprofilen, also zwischen dem ersten Abgabe-Tagesprofil und dem ersten weiteren Abgabe-Tagesprofil und/oder zwischen zwei direkt aufeinander folgenden ansprüchsgemässen weiteren Abgabe-Tagesprofilen, einem Zeitbetrag entspricht, der durch ganzzahlige Teilung der zur Ermittlung mindestens eines dieser Abgabe-Tagesprofile herangezogenen ersten, zweiten oder korrigierten zweiten Zeitverschiebung erhalten wurde, wobei es bevorzugt ist, wenn dieser Zeitbetrag kleiner oder gleich 4 Stunden ist, bevorzugterweise kleiner oder gleich 3 Stunden.

Sind dabei die Zeitverschiebungsdifferenzen zwischen allen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, identisch, was bevorzugt ist, so lässt sich die Anpassung auch an grosse Zeitverschiebungen in einheitlichen Schritten vollziehen, ohne dass übermässige Korrektursprünge entstehen.

In einer bevorzugten Ausführung des erfindungsgemässen Verfahrens, bei welcher ein Teil oder alle der weiteren Abgabe-Tagesprofile einem zeitlich um eine bestimmte Zeitverschiebung verschobenen ersten Abgabe-Tagesprofil entsprechen, wird eine gewünschte oder maximal zulässige Zeitverschiebungsdifferenz zwischen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, der Steuerung durch manuelle Eingabe oder über eine insbesondere drahtlose Schnittstelle mitgeteilt oder ist bereits in der Steuerung abgespeichert. Sodann werden die weiteren Abgabe-Tagesprofile mit der Steuerung in Abhängigkeit von der ersten, der zweiten oder der korrigierten zweiten Zeitverschiebung und von der gewünschten oder zulässigen Zeitverschiebungsdifferenz ermittelt.

Auch ist bei einer Ausführung der erfindungsgemässen Dosiervorrichtung, bei welcher die Steuerung derartig ausgestaltet ist, dass mit ihr weitere Abgabe-Tagesprofile ermittelbar und zur automatischen Steuerung verwendbar sind, welche einem zeitlich um eine bestimmte Zeitverschiebung verschobenen ersten Abgabe-Tagesprofil entsprechen, bevorzugt, wenn die Steuerung zudem derartig ausgestaltet ist, dass eine gewünschte oder maximal zulässige Zeitverschiebungsdifferenz zwischen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, der Steuerung durch manuelle Eingabe oder über eine insbesondere drahtlose Schnittstelle mitgeteilt werden kann oder in der Steuerung abgespeichert ist und mit der Steuerung in Abhängigkeit von der ersten oder der zweiten Zeitverschiebung und von der gewünschten oder zulässigen Zeitverschiebungsdifferenz die weiteren Abgabe-Tagesprofile ermittelbar sind. Hierdurch wird es möglich, in einfacher Weise auf den Algorithmus zur Ermittlung der weiteren Abgabe-Tagesprofile Einfluss zu nehmen, insbesondere bezüglich der zugrunde zu legenden Anpassungsgeschwindigkeit.

In einer weiteren bevorzugten Ausführungsform der Erfindung entspricht zumindest ein Teil der ermittelten weiteren Abgabe-Tagesprofile einem im wesentlichen stufenlos oder an einer oder an mehreren Stellen zeitlich gestauchten und/oder gedehnten ersten Abgabe-Tagesprofil. Auf diese Weise können Abgabe-Tagesprofile ermittelt und zur Steuerung der Dosiervorrichtung verwendet werden, welche optimal an das jeweilige physiologische Bedarfsprofil des Patienten angepasst sind.

Dabei ist es bevorzugt, wenn diese weiteren Abgabe-Tagesprofile intervallweise Dehnungen und/oder Stauchungen um jeweils einen bestimmten Zeitbetrag aufwiesen, bevorzugterweise jede Stunde, alle 2 Stunden, alle 4 Stunden oder alle 6 Stunden eine Dehnung oder Stauchung um einen bestimmten Zeitbetrag aufweisen. Dabei ist es je nach Anwendungsfall entweder bevorzugt, wenn der Zeitbetrag bei allen Dehnungen und/oder Stauchungen identisch ist oder aber wenn die Dehnungen und/oder Stauchungen über das Abgabe-Tagesprofil gesehen verschiedene Zeitbeträge aufweisen.

Zudem ist es bei diesen Ausführungsformen der Erfindung bevorzugt, wenn alle der ermittelten gedehnten und/oder gestauchten Abgabe-Tagesprofile eine identische Profilform aufweisen und zeitlich zueinander verschoben sind.

Durch die letztgenannten Massnahmen lässt sich sicherstellen, dass eine gute Anpassung an das jeweilige physiologische Bedarfsprofil möglich ist und gleichzeitig die schrittweisen Anpassungen an den Abgabe-Tagesprofilen überschaubar bleiben, was im Lichte einer einfachen Überprüfbarkeit der korrekten Funktion der Dosiervorrichtung von Vorteil ist.

In einer bevorzugten Ausführungsform des erfindungsgemässen Verfahrens wird der Steuerung ein gewünschter oder maximal zulässiger Zeitbetrag je Dehnung und/oder Stauchung und/oder eine gewünschte oder maximal und/oder minimal zulässige Anzahl von Dehn- und/oder Stauchschritten und/oder ein gewünschtes oder maximal und/oder minimal zulässiges Intervall zwischen den Dehn- und/oder Stauchschritten durch manuelle Eingabe oder über eine bevorzugterweise drahtlose Schnittstelle mitgeteilt oder ist bereits in der Steuerung abgespeichert. Sodann werden mit der Steuerung automatisch in Abhängigkeit von der ersten Zeitverschiebung und von dem gewünschten oder zulässigen Zeitbetrag je Dehnung und/oder Stauchung und/oder von der gewünschten oder maximal und/oder minimal zulässigen Anzahl von Dehn- und/oder Stauchschritten und/oder von dem gewünschten oder maximal und/oder minimal zulässigen Intervall zwischen den Dehn- und/oder Stauchschritten die weiteren Abgabe-Tagesprofile ermittelt.

In einer bevorzugten Ausführungsform der Dosiervorrichtung ist die Steuerung derartig ausgestaltet, dass ein gewünschter oder maximal zulässiger Zeitbetrag je Dehnung und/oder Stauchung und/oder eine gewünschte oder maximal und/oder minimal' zulässige Anzahl von Dehn- und/oder Stauchschritten und/oder ein gewünschtes oder maximal und/oder minimal zulässiges Intervall zwischen den Dehn- und/oder Stauchschritten der Steuerung durch manuelle Eingabe oder über eine insbesondere drahtlose Schnittstelle mitgeteilt werden kann oder in der Steuerung abgespeichert ist. Zudem ist die Steuerung derartig ausgestaltet, dass mit ihr automatisch in Abhängigkeit von der ersten oder zweiten Zeitverschiebung und von dem gewünschten oder zulässigen Zeitbetrag je Dehnung und/oder Stauchung und/oder von der gewünschten oder maximal und/oder minimal zulässigen Anzahl von Dehn- und/oder Stauchschritten und/oder von dem gewünschten oder maximal und/oder minimal zulässigen Intervall zwischen den Dehn- und/oder Stauchschritten die weiteren Abgabe-Tagesprofile ermittelbar sind.

In noch einer weiteren bevorzugten Ausführungsform der Erfindung ist es bevorzugt, wenn zumindest ein Teil der mit der Steuerung ermittelten weiteren Abgabe-Tagesprofile einem zeitlich um eine bestimmte Zeitverschiebung verschobenen und zugleich zeitlich gestauchten oder zeitlich gedehnten ersten Abgabe-Tagesprofil entspricht.

Hierdurch wird es möglich, die Betriebsweise der Dosiervorrichtung optimal an die jeweiligen Gegebenheiten anzupassen.

In noch einer weiteren bevorzugten Ausführungsform der Erfindung entspricht ein auf ein mit der Steuerung ermitteltes anspruchsgemässes weiteres Abgabe-Tagesprofil folgendes Abgabe-Tagesprofile dem um die erste Zeitverschiebung oder um die Summe aus erster und zweiter Zeitverschiebung verschobenen ersten Abgabe-Tagesprofil, was bedeutet, dass die Anpassung an die erste oder die erste und die zweite Zeitverschiebung vollständig abgeschlossen ist.

Mit Vorteil werden die jeweiligen Zeitverschiebungen, welche von der Steuerung zur Ermittlung der weiteren Abgabe-Tagesprofile berücksichtigt werden, der Dosiervorrichtung bzw. deren Steuerung durch manuelle Eingabe, z.B. über eine Tastatur, oder durch Datenübertragung über eine drahtgebundene oder mit Vorteil drahtlose Schnittstelle, z.B. über eine Funkverbindung mittels eines separaten Programmiergerätes, mitgeteilt. Auch ist es bevorzugt, die Zeitverschiebungen mit der Steuerung durch Auswertung bevorzugterweise drahtlos empfangener Daten zu ermitteln, vorteilhafterweise mittels eines in der Dosiervorrichtung integrierten Satelliten-Navigationssystems, wie beispielsweise eines GPS-Systems.

Auch ist es bevorzugt, wenn die computergestützte Steuerung der Dosiervorrichtung mindestens zwei getrennte, bevorzugterweise gemeinsam getaktete Uhren aufweist, von denen mit einer ersten die erste Zeitverschiebung oder die Summe der Zeitverschiebungen oder eine diese Zeitverschiebungen repräsentierende Uhrzeit verwaltet wird, im Falle von reisebedingten Zeitverschiebungen die zweite oder die dritte Ortszeit, wobei diese zudem bevorzugterweise über ein Display an der Dosiervorrichtung angezeigt wird. Die zweite der Uhren wird zur Steuerung der Medikamentenförderung der Dosiervorrichtung gemäss einem Abgabe-Tagesprofil verwendet. Dabei wird die zweite der Uhren bei einer Zeitverschiebung zur Steuerung der Dosiervorrichtung gemäss einem mit der Steuerung ermittelten weiteren Abgabe-Tagesprofil über die Steuerung automatisch derartig eingestellt, dass sie einen Zeitversatz gegenüber einer Basiszeit vor der ersten Zeitverschiebung, bei reisebedingten Zeitverschiebungen ist dies die anspruchsgemässe erste Ortszeit, aufweist, der kleiner oder gleich der ersten Zeitverschiebung oder der Summe aus erster und zweiter Zeitverschiebung ist.

Dabei ist es bei Ausführungsformen der Erfindung, bei denen die mit der Steuerung ermittelten weiteren Abgabe-Tagesprofile einem zeitlich verschobenen ersten Abgabe-Tagesprofil entsprechen, von Vorteil, wenn die zweite Uhr über die Steuerung automatisch derartig eingestellt wird, dass sie jeweils einen Zeitversatz gegenüber der Basiszeit bzw. der ersten Ortszeit aufweist, welcher der Zeitverschiebung des gerade zur Steuerung der Dosiervorrichtung verwendeten weiteren Abgabe-Tagesprofils gegenüber dem ersten Abgabe-Tagesprofil entspricht.

Auf diese Weise lassen sich einfach aufgebaute, zuverlässige erfindungsgemässe Dosiervorrichtungen bereitstellen, welche auf sichere und überschaubare Weise gemäss dem erfindungsgemässen Verfahren betrieben werden können.

In noch einer weiteren bevorzugten Ausführungsform der Erfindung wird der Grad der Anpassung des Betriebs der Dosiervorrichtung an die erste oder zweite Zeitverschiebung bzw. bei reisebedingter Zeitverschiebung der Grad der Umstellung des Betriebs der Dosiervorrichtung auf die zweite Ortszeit oder auf die dritte Ortszeit, oder mit anderen Worten gesagt der Grad der Abweichung des aktuell zur Steuerung verwendeten weiteren Abgabe-Tagesprofils von dem nach vollständiger Anpassung oder Umstellung zum weiteren Betrieb der Dosiervorrichtung vorgesehenen Abgabe-Tagesprofil, welches üblicherweise dem um die erste Zeitverschiebung oder um die Summe aus erster und zweiter Zeitverschiebung verschobenen ersten Abgabe-Tagesprofils entspricht, an der Dosiervorrichtung graphisch dargestellt. Dieses erfolgt mit Vorteil derart, das die Abstände zwischen dargestellten graphischen Elementen und/oder die Erstreckung solcher graphischer Elemente zur Darstellung von Zeitdifferenzen verwendet werden.

Dabei ist es bevorzugt, wenn das Verhältnis der Basiszeit, einer um die erste Zeitverschiebung oder die Summe aus erster und zweiter Zeitverschiebung gegenüber der Basiszeit verschobenen Zeit sowie der aktuelle Grad der Anpassung des Betriebs der Dosiervorrichtung auf die erste oder zweite Zeitverschiebung oder, in den zuvor für den Fall einer reisebedingter Zeitverschiebung verwendeten Begriffen ausgedrückt, das Verhältnis der ersten Ortszeit, der zweiten Ortszeit und/oder der dritten Ortszeit sowie der aktuelle Grad der Umstellung des Betriebs der Dosiervorrichtung auf die zweite oder die dritte Ortszeit, vorteilhafterweise durch Piktogramme, deren Abstände zueinander der Darstellung der jeweiligen Zeitdifferenzen dienen und insbesondere proportional zu den jeweiligen Zeitdifferenzen sind, grafisch dargestellt wird.

Auch ist es bevorzugt, wenn der aktuelle Grad der Anpassung des Betriebs der Dosiervorrichtung an die erste oder zweite Zeitverschiebung oder, bei reisebedingter Zeitverschiebung, die Umstellung des Betriebs der Dosiervorrichtung auf die zweite oder dritte Ortszeit dadurch dargestellt wird, dass die aktuelle Zeit der zweiten Uhr angezeigt und/oder im Verhältnis zu einer um die erste Zeitverschiebung oder die Summe aus erster und zweiter Zeitverschiebung gegenüber der Basiszeit verschobenen Zeit oder, bei reisebedingter Zeitverschiebung, im Verhältnis zur zweiten oder dritten Ortszeit dargestellt wird, und bevorzugterweise zusätzlich zur Basiszeit bzw. zur ersten Ortszeit. Auf diese Weise ist die aktuelle Betriebsituation der Dosiervorrichtung jederzeit auf einen Blick erfassbar, was die Kontrolle der korrekten Funktion derselben erleichtert.

In noch einer weiteren bevorzugten Ausführungsform der Erfindung ist die um die erste Zeitverschiebung oder die Summe aus erster und zweiter Zeitverschiebung gegenüber der Basiszeit verschobene Zeit bzw. bei reisebedingter Zeitverschiebung die zweite und/oder die dritte Ortszeit und bevorzugterweise zusätzlich die Basiszeit bzw. die erste Ortszeit an der Dosiervorrichtung als analoge oder digitale Uhrenanzeige anzeigbar.

Für die zuvor erwähnten Massnahmen zur Anzeige von Zeiten und Zeitverhältnissen verfügt die Dosiervorrichtung über geeignete Anzeigemittel, wie beispielsweise einen LCD-Display mit einer entsprechenden Graphiksoftware, welche mit der Steuerung in geeigneter Weise wirkverbunden sind.

In noch einer weiteren bevorzugten Ausführungsform der erfindungsgemässen Dosiervorrichtung besteht die Möglichkeit, mit der Dosiervorrichtung zusätzlich Boli zu berechnen, im Falle einer Insulintherapie beispielsweise Korrekturboli oder Mahlzeitenboli, und bei der Berechnung automatisch Korrekturen in Abhängigkeit vom Grad der Anpassung des Betriebs der Dosiervorrichtung an die erste oder zweite Zeitverschiebung oder, bei reisebedingten Zeitverschiebungen, vom aktuellen Grad der Umstellung des Betriebs der Dosiervorrichtung auf die zweite oder die dritte Ortszeit zu berücksichtigen, was gemäss einer bevorzugten Ausführungsform des erfindungsgemässen Verfahrens erfolgt.

Dabei ist es bevorzugt, dass die Korrekturen derartig durchführbar sind bzw. vorgenommen werden, dass in der Umstellungsphase vorübergehend eine grössere Abweichung vom sonst üblichen Zielwert oder Zielwertkorridor eines Parameters zugelassen wird, im Falle einer Insulintherapie beispielsweise vorübergehend eine grössere Abweichung der Blutglucosewerte zugelassen wird.

In noch einer weiteren bevorzugten Ausführungsform können Steuerungsdaten der Dosiervorrichtung als Steuerungs-Historie mit Referenz auf die Basiszeit oder, bei reisebedingter Zeitverschiebung, mit Referenz auf die erste Ortszeit abgespeichert werden. Dies weist gegenüber der natürlich ebenfalls denkbaren Abspeicherung der Historie mit Referenz zur um die erste Zeitverschiebung oder die Summe aus erster und zweiter Zeitverschiebung gegenüber der Basiszeit verschobenen Zeit bzw. zur zweiten oder dritten Ortszeit den Vorteil auf, dass die Basiszeit bzw. die erste Ortszeit als einzige Zeit von der Zeitverschiebung überhaupt nicht betroffen ist. Bevorzugterweise können dabei zumindest bei der Speicherung der Daten von therapierelevanten Informationen, z.B. von Bolusgaben, zusätzlich die um die erste Zeitverschiebung oder um die Summe aus erster und zweiter Zeitverschiebung gegenüber der Basiszeit verschobene Zeit bzw. bei reisebedingter Zeitverschiebung die zweite oder die dritte Ortszeit abgespeichert werden, was gemäss einer bevorzugten Ausführungsform des erfindungsgemässen Verfahrens erfolgt.

Ein dritter Aspekt der Erfindung betrifft die Anwendung des erfindungsgemässen Verfahrens gemäss dem ersten Aspekt der Erfindung zum Betrieb einer computergesteuerten Insulinpumpe, insbesondere zur Zuführung von Insulin in den Körper eines Patienten.

Ein vierter Aspekt der Erfindung betrifft die Verwendung der Dosiervorrichtung gemäss dem zweiten Aspekt der Erfindung zur dosierten Förderung von Insulin, insbesondere in den Körper eines Patienten.

Bei solchen Anwendungen und Verwendungen kommen die Vorteile der Erfindung besonders deutlich zum Tragen.

Weitere bevorzugte Ausführungen der Erfindung ergeben sich aus den abhängigen Ansprüchen und aus der nun folgenden Beschreibung anhand der Figuren. Dabei zeigen:
Fig. 1 ein typisches basales Abgabe-Tagesprofil einer computergesteuerten Insulinpumpe;
die Figuren 2a bis 2c das Abgabe-Tagesprofil aus Fig. 1 im Verhältnis zu zwei um verschiedene Zeitverschiebungen verschobenen Abgabe-Tagesprofilen;
die Figuren 3a bis 3c das Abgabe-Tagesprofil aus Fig. 1 bei stufenweiser Anpassung an eine Zeitverschiebung;
Fig. 4 schematisch die Architektur der Steuerung einer erfindungsgemässen Dosiervorrichtung;
die Figuren 5a bis 5c die Anzeigen auf dem Display einer erfindungsgemässen Dosiervorrichtung während verschiedener Stadien der Anpassung an eine Zeitverschiebung; und
Fig. 6 eine Display-Anzeige wie bei Fig. 5a bei umgekehrter Zeitverschiebung.

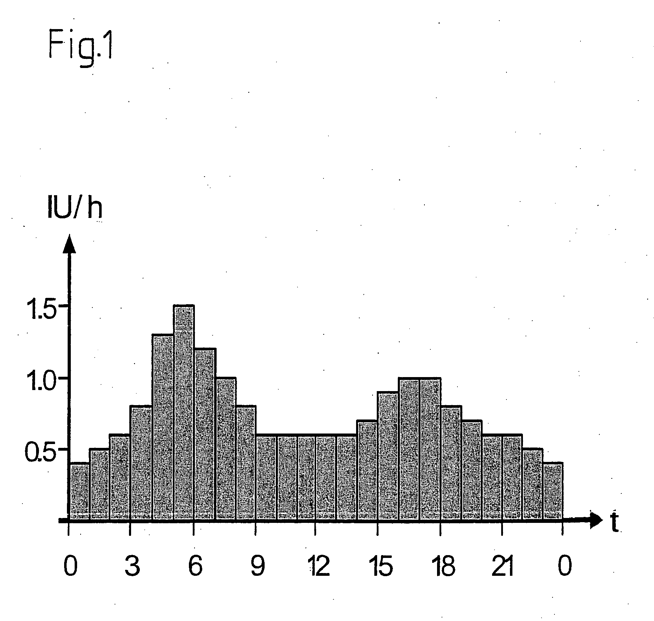

Ein typisches Abgabe-Tagesprofil einer computergesteuerten, am Körper tragbaren Insulinpumpe, auch als basales Förderprofil der Insulinpumpe bezeichnet, ist in Fig. 1 dargestellt, welches der Grundversorgung des Körpers eines Diabetikers mit Insulin dient. Auf der Ordinate ist dabei die Förderrate in Insulineinheiten pro Stunde IU/h aufgetragen, auf der Abszisse die Zeit t im Umfang eines Wochentags mit 24 Stunden. Wie zu erkennen ist, wird durch die häufige subkutane Abgabe kleinster Insulinmengen in den Körper des Diabetikers eine kontinuierliche Versorgung mit Insulin angenähert, welche dem physiologischen Insulinbedarf des Körpers entspricht. Dabei weist das typische Abgabeprofil ein erstes Maximum in den frühen Morgenstunden auf, das sogenannte "Dämmerungsphenomen", und ein zweites Maximum am späten Nachmittag, während um Mitternacht herum ein Bedarfsminimum vorliegt.

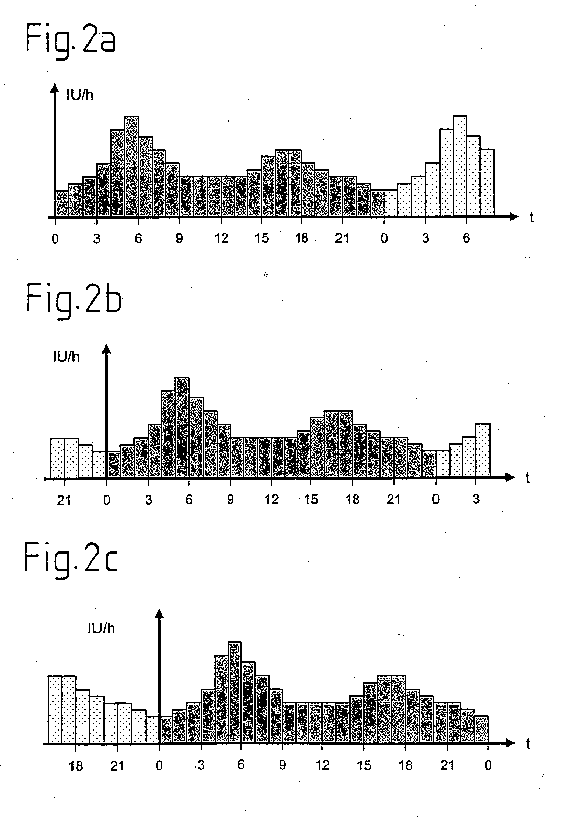

Die Figuren 2a bis 2c zeigen das Abgabe-Tagesprofil aus Fig. 1, einmal zur Ortszeit am Heimatort (Fig. 2b), einmal zeitlich verschoben um eine Zeitverschiebung von plus 4 Stunden (Fig. 2a), wie sie z.B. bei einer Reise in Richtung Osten auftreten kann, und einmal zeitlich verschoben um eine Zeitverschiebung von minus 4 Stunden (Fig. 2c), wie sie z.B. bei einer Reise in Richtung Westen auftreten kann, im Verhältnis zueinander. Wie zu erkennen ist, kommt es bereits bei einer Zeitverschiebung von einigen Stunden bezogen auf das am Heimatort verwendete Abgabe-Tagesprofil zu einer massiven Umstellung der einzelnen Förderraten, weshalb von den meisten Ärzten empfohlen wird, Anpassungen an Zeitverschiebungen grösser 2 Stunden schrittweise vorzunehmen.

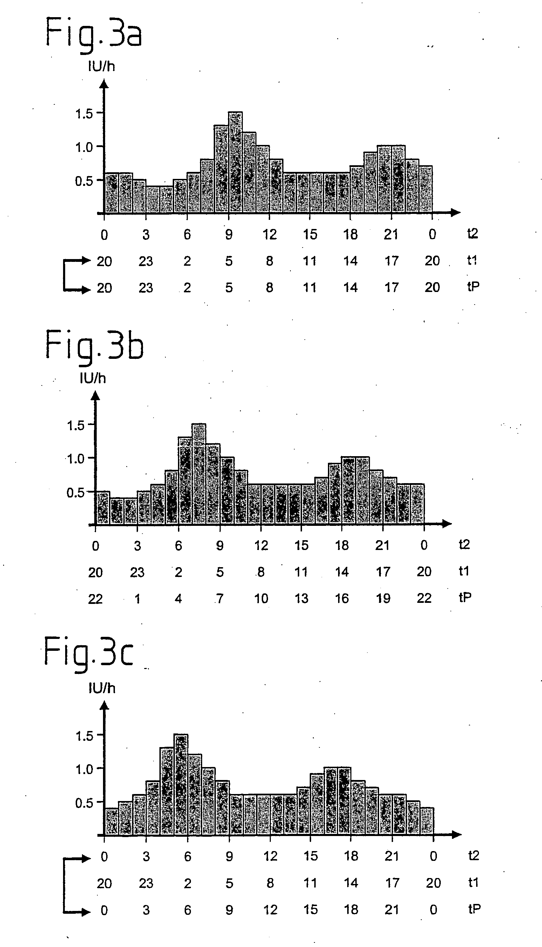

Eine solche schrittweise Umstellung des Abgabe-Tagesprofils einer computergestützten Insulinpumpe von der Ortszeit t1 am Heimatort auf eine gegenüber der Heimatzeit t1 um 4 Stunden vorverschobene Ortszeit t2 am Zielort einer Reise, also beispielsweise eine stufenweise Umstellung von dem in Fig. 2b gezeigten Abgabe-Tagesprofil am Heimatort auf das in Fig. 2a gezeigte Abgabe-Tagesprofil am Zielort, welches dem um eine Zeitverschiebung von 4 Stunden verschobenen Abgabe-Tagesprofil am Heimatort entspricht, ist in den Figuren 3a bis 3c dargestellt. Wie zu erkennen ist, erfolgt hier die Anpassung des Förderprofils der Insulinpumpe durch stufenweises Umstellen der von der Pumpenuhr verwalteten Pumpenzeit tP, welche vor der Anpassung identisch mit der Heimatzeit t1 ist (siehe die zwei verbundenen Pfeile in Fig. 3a), um täglich 2 Stunden (Zwischenschritt siehe Fig. 3b), bis die Pumpenzeit tP identisch mit der Ortszeit t2 am Zielort ist (siehe die zwei verbundenen Pfeile in Fig. 3c). Diese stufenweise Anpassung (Verschiebung) des basalen Abgabe-Tagesprofils wird bei der hier verwendeten erfindungsgemässen Insulinpumpe von der Steuerung in Abhängigkeit von der Zeitverschiebung zwischen dem Heimatort und dem Zielort der Reise automatisch ermittelt und zur Steuerung der Insulinförderung verwendet. Hierbei wird die Umstellungshandlung durch den Insulinpumpenträger zu einem einzigen Zeitpunkt vorgenommen, z.B. bereits vor der Reise, während der Reise oder zum Zeitpunkt der Ankunft am Zielort. Der Benutzer gibt dabei entweder die neue Ortszeit t2 am Zielort oder aber die Zeitverschiebung zwischen Heimatzeit t1 und Zielzeit t2 an. Zudem gibt der Benutzer der Pumpensteuerung an, in welcher zeitlicher Abstufung bzw. mit welcher Anpassungsgeschwindigkeit die Anpassung des Basalprofils erfolgen soll, im vorliegenden Fall um zwei Stunden pro Tag.

Zur Realisierung der Zeitverschiebung werden bei der hier verwendeten erfindungsgemässen Insulinpumpe nicht die einzelnen Basalraten verschoben, sondern die Steuerung der basalen Ausschüttung erfolgt pumpenintern durch eine getrennte Uhr, welche die Pumpenzeit tP verwaltet, welche gegenüber der Ortszeit t2 am Zielort, die auch an einem Display an der Insulinpumpe angezeigt wird, einen variablen Zeitversatz Δ*t* aufweist, der zu jedem Zeitpunkt der Verschiebung zwischen der Ortszeit t2 und dem gerade zur Steuerung der Insulinausschüttung verwendeten Abgabe-Tagesprofil entspricht.

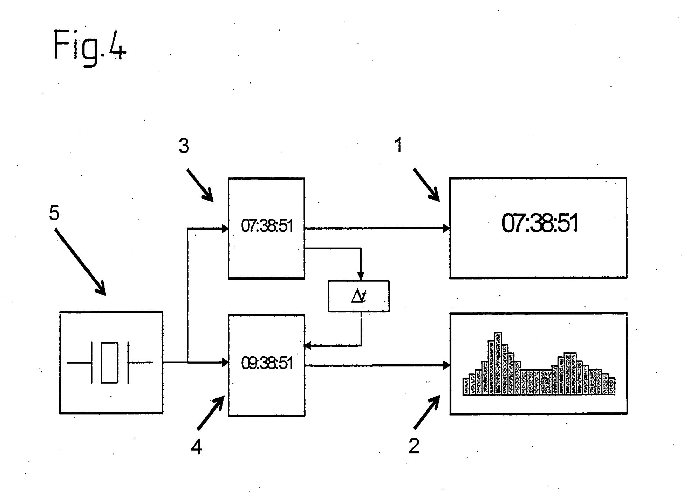

Wie aus Fig. 4 erkennbar ist, welche schematisch die Architektur der Steuerung der verwendeten Insulinpumpe zeigt, werden die Uhrzeitanzeige 1 und die Fördereinrichtung 2 der Insulinpumpe über getrennte Uhren 3, 4 gesteuert, die einen gemeinsamen Taktgenerator 5 aufweisen. Die erste Uhr 3 ist dabei die zentrale Hauptuhr der Pumpensteuerung, die, wie bereits erwähnt wurde, die neue Ortszeit t2 verwaltet, die auch im Pumpendisplay 1 angezeigt wird. Sie wird z.B. bei Beginn oder Ende der Reise auf die neue Ortszeit t2 eingestellt. Die zweite Uhr 4 dient der Steuerung der Insulinförderung über die von ihr verwaltete Pumpenzeit tP, welche über die Steuerung schrittweise auf die neue Ortszeit t2 (erste Uhr 3) umgestellt wird. Die Gesamtsteuerung der Pumpe kann grundsätzlich wahlweise über eine der beiden Uhren 3, 4 erfolgen. Im vorliegenden Fall erfolgt sie jedoch über die erste Uhr 3, was vorteilhaft ist, da diese von den wenigsten Umstellungen betroffen ist (nur je eine Umstellung für Hin- und Rückreise). Aus diesem Grund erfolgen auch die Eintragungen in die Pumpenhistory bevorzugt mit Referenz auf die erste Uhr 3. Zur Sicherstellung der korrekten Funktion bzw. zur Fehlerdetektion wird die jeweils aktuelle Zeitverschiebung Δ*t* der Zeit tP der zweiten Uhr 4 gegenüber der Zeit t2 der ersten Uhr 3 zu festgelegten Zeitpunkten überprüft, z.B. bei jeder basalen Insulinausschüttung, stündlich, oder bei jeder Änderung von Δ*t*, was im vorliegenden Fall automatisch durch die Pumpensteuerung erfolgt.

Wie aus den Figuren 5a bis 5c hervorgeht, welche die Anzeigen auf dem Display einer weiteren erfindungsgemässen Insulinpumpe während einer mehrstufigen Anpassung des Abgabe-Tagesprofils an eine mehrstündige reisebedingte Zeitverschiebung zeigen, besteht bei der verwendeten Insulinpumpe die Möglichkeit, die Ortszeiten am Heimatort und am Zielort sowie die dem aktuell verwendeten Abgabe-Tagesprofils zugrunde liegende Zeitverschiebung gegenüber diesen Ortszeiten in Relation zueinander darzustellen, derart, dass diese durch Piktogramme ("Haus" für Ortszeit am Heimatort; "Ausschüttungsprofil" für aktuelle Zeitverschiebung des gerade verwendeten Abgabe-Tagesprofils; "Uhr" für Ortszeit am Zielort) dargestellt werden. "Haus" und "Uhr" sind dabei so angeordnet, dass sie die Displaybreite nahezu ausnutzen. Bei der hier vorliegenden positiven Zeitverschiebung ist das Piktogramm "Uhr" rechts und das Piktogramm "Haus" links im Display angezeigt. Das Piktogramm "Ausschüttungsprofil" ist dazwischen eingezeichnet, und zwar so, dass die Abstände a zum "Haus" (Ortszeit am Heimatort) und b zur "Uhr" (Ortszeit am Zielort) im Rahmen der Displayauflösung proportional zu den jeweiligen Zeitverschiebungen gegenüber diesen Ortszeiten sind. Insbesondere entspricht b der aktuellen Zeitverschiebung Δ*t* der Pumpenzeit gegenüber der Ortszeit am Zielort oder mit anderen Worten gesagt, der Zeitverschiebungsdifferenz zwischen der Zeitverschiebung zwischen Heimat- und Zielort und der aktuellen Zeitverschiebung des gerade zur Insulinförderung verwendeten Abgabe-Tagesprofils gegenüber dem zuvor am Heimatort verwendeten Abgabe-Tagesprofil.

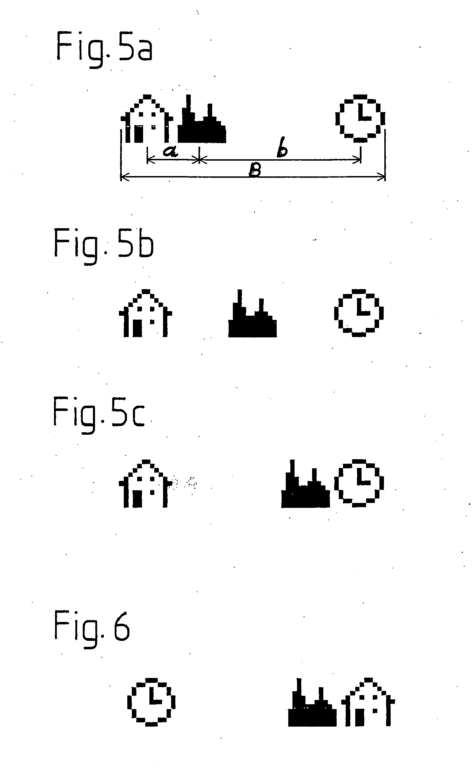

Dabei zeigen Fig. 5a die Situation kurz nach Beginn der Umstellung der Pumpenuhr auf die Ortszeit am Zielort (die basale Insulinabgabe richtet sich noch weitgehend nach der Heimatzeit), Fig. 5b die Situation mitten während der Umstellungsphase und Fig. 5c die Situation am Ende der Umstellungsphase (die basale Insulinabgabe richtet sich schon weitgehend nach der Ortszeit am Zielort). Das Piktogramm "Ausschüttungsprofil" wandert demnach während der Umstellungsphase vom Piktogramm "Haus" (Ortszeit am Heimatort) zum Piktogramm "Uhr" (Ortszeit am Zielort).

Fig. 6 zeigt zur Verdeutlichung die zu Fig. 5a analoge Darstellung bei einer Reise in Richtung Westen (negative Zeitverschiebung).

Erfolgt die Anpassung der Insulinpumpe an die Zeitverschiebung in relativ feinen zeitlichen Abstufungen (z.B. stündlich), so kommt es am Anfang und gegen Ende der Umstellung zu einer Überlappung des Piktogramms "Ausschüttungsprofil" mit den Piktogrammen "Haus" bzw. "Uhr". In diesem Fall können die überlappenden Piktogramme z.B. alternierend blinkend dargestellt werden. Grundsätzlich vermeiden lässt sich die Überlappung, indem das bewegliche Piktogramm "Ausschüttungsprofil" vertikal versetzt, ober- oder unterhalb der Piktogramme "Haus" und "Uhr" angeordnet wird.

Wahlweise können, z.B. ober- oder unterhalb der jeweiligen Piktogramme, zusätzlich die Ortszeiten am Heimatort und am Zielort angezeigt werden, wodurch gleichzeitig eine Uhr mit zwei Zeitzonen realisiert wird. Zahlreiche weitere Varianten zur Visualisierung der Zeitverschiebungen sind ebenfalls denkbar.

Da häufig mit einem bestimmten Abstand voneinander zwei Zeitverschiebungen um den gleichen Betrag aber mit entgegengesetztem Vorzeichen vorgenommen werden müssen, z.B. bei einer Urlaubshin- und Rückreise, verfügt die Insulinpumpe zudem über einen speziellen "Rückreise-Befehl", durch den die stufenweise Zeitverschiebung der Rückreise automatisch durchgeführt wird, ohne dass die Zeitverschiebung neu programmiert werden muss. Treten während der Reise selbst weitere Zeitverschiebungen auf, z.B. bei grossen Urlaubsreisen mit Zwischenaufenthalten, welche zudem in beiden Richtungen möglich sind, so werden alle während der Reise auftretenden Zeitverschiebungen vorzeichenrichtig aufsummiert und durch den "Rückreise-Befehl" gemeinsam rückgängig gemacht, so dass hierdurch automatisch wieder eine Umstellung auf die Heimatzeit erfolgt.

Speziell bei mehreren aufeinander folgenden Zeitverschiebungen ergibt es sich oftmals, dass sie sich überlagern, d.h. die jeweils nächste Zeitverschiebung erfolgt, bevor die Anpassung an die vorherige Zeitverschiebung abgeschlossen ist. Diese Überlagerung wird automatisch bei der darauf folgenden Zeitverschiebung berücksichtigt. Ist z.B. eine Umstellung auf eine Zeitverschiebung von plus 4 Stunden (Reise in Richtung Osten) bereits zur Hälfte erfolgt (Δ*t* = 2 Stunden) und durch die Weiterreise ergibt sich eine weitere Zeitverschiebung von plus 1 Stunde, so ist noch eine Umstellung auf eine Zeitverschiebung von plus 3 Stunden vorzunehmen.

Des Weiteren bietet die hier verwendete erfindungsgemässe Insulinpumpe die Möglichkeit, die Berechnung von Mahlzeiten- und Korrekturboli automatisch vorzunehmen. Die hierfür erforderlichen Umrechnungskonstanten sind typischerweise uhrzeitabhängig, folgen also ebenso wie der basale Insulinbedarf einem tageszeitabhängigen Profil. Ihre Anpassung bei einem Zeitzonenwechsel erfolgt hier deshalb ebenfalls schrittweise, jeweils gemeinsam mit der Verschiebung des basalen Abgabeprofils. Da im Anschluss an eine längere Reise für eine gewisse Übergangsphase mit einer schlechteren Stoffwechseleinstellung zu rechnen ist, die jedoch für eine begrenzte Zeit toleriert werden kann, sollten in dieser Phase Korrekturen in beiden Richtungen (insbesondere durch zusätzliche Insulingaben oder die Einnahme von Glukose) nur behutsam durchgeführt werden, da zu häufige und heftige Korrekturen zu einer Instabilität des Stoffwechsels führen können. Im Rahmen der automatischen Berechnung der Boli wird daher der durch die Berechnung angestrebte "Zielkorridor" der Blutglucose für eine begrenzte Zeit weiter gefasst, d.h. es werden vorübergehend bewusst höhere und ggf. auch tiefere Blutglucosewerte als ansonsten üblich ohne Korrektur zugelassen. Die Rückführung auf die Ursprungswerte erfolgt hier automatisch in Abhängigkeit von bestimmten abgespeicherten Parametern, wie z.B. der Gesamtdauer der Reise, entweder in einem Schritt, z.B. am Ende der Zeitumstellungsphase, oder ebenfalls schrittweise.

Des Weiteren weist die verwendete erfindungsgemässe Insulinpumpe für die Programmierung von reisebedingten Zeitverschiebungen ein spezielles "Reisemenü" auf, in welchem in Abhängigkeit von der eingegebenen Zeitverschiebung bestimmte Eingaben abgefragt werden. Bei einer Umstellung um kleine Beträge (z.B. Zeitverschiebungen bis 2h), wie sie bei einer gelegentlichen Korrektur des Fehlgangs oder bei der Sommerzeitumstellung erfolgen, wird die Umstellung ohne stufenweise Anpassung vorgenommen. Bei einer Umstellung um einen grösseren Betrag erfolgt automatisch eine Abfrage, ob (und ggf. mit welchen Parametern) eine schrittweise Anpassung der basalen Insulinabgabe erfolgen soll. Da eine reisebedingte Zeitumstellung praktisch der einzige Grund für grosse Uhrzeitumstellungen im laufenden Betrieb sind, ist es auch vorgesehen, die hier verwendete Funktionalität unmittelbar in die Uhrzeitumstellung zu integrieren. Dabei kann die Differenz zwischen der Ortszeit am Heimatort und der Ortszeit am Zielort gespeichert werden, um die Zeitverschiebung mit einem "Undo-Befehl" Rückgängig machen zu können. Dies entspricht dem zuvor erwähnten "Rückreise-Befehl". Dieser Befehl kann auch zur einfachen Umstellung der Pumpenuhr zwischen Sommer- und Winterzeit genutzt werden.

## Patentansprüche

1. Verfahren zum Betrieb einer Dosiervorrichtung für flüssige Medikamente mit einer computergestützten Steuerung bei reisebedingter Verschiebung der Ortszeit, umfassend die Schritte:
a) Steuern der Dosiervorrichtung mit der Steuerung an einem ersten Ort mit einer ersten Ortszeit (t1) derart, dass diese das flüssige Medikament automatisch gemäss einem ersten Abgabe-Tagesprofil abgibt;
b) Bringen der Dosiervorrichtung an einen zweiten Ort mit einer zweiten Ortszeit (t2), welche gegenüber der ersten Ortszeit (t1) um eine erste Zeitverschiebung verschoben ist;
c) Steuern der Dosiervorrichtung mit der Steuerung direkt im Anschluss an die Abgabe gemäss dem ersten Abgabe-Tagesprofil vorübergehend derart, dass diese das flüssige Medikament automatisch gemäss mindestens einem weiteren Abgabe-Tagesprofil abgibt, welches von dem ersten Abgabe-Tagesprofil und dem um die erste Zeitverschiebung verschobenen ersten Abgabe-Tagesprofil verschieden ist,
wobei mit der Steuerung das mindestens eine weitere Abgabe-Tagesprofil in Abhängigkeit von der ersten Zeitverschiebung ermittelt wird.

2. Verfahren nach Anspruch 1, wobei die Dosiervorrichtung nach dem zweiten Ort an einen dritten Ort mit einer dritten Ortszeit, welche gegenüber der zweiten Ortszeit (t2) um eine zweite Zeitverschiebung verschoben ist, gebracht wird und die Dosiervorrichtung mit der Steuerung vorübergehend derartig gesteuert wird, dass diese das flüssige Medikament automatisch gemäss mindestens einem weiteren Abgabe-Tagesprofil abgibt, welches von dem zuletzt am zweiten Ort verwendeten Abgabe-Tagesprofil und dem um die vorzeichenrichtige Summe aus der ersten und der zweiten Zeitverschiebung verschobenen ersten Abgabe-Tagesprofil verschieden ist, wobei dieses mindestens eine weitere Abgabe-Tagesprofil mit der Steuerung in Abhängigkeit von der zweiten Zeitverschiebung ermittelt wird.

3. Verfahren nach Anspruch 2, wobei das zuletzt am zweiten Ort verwendete weitere Abgabe-Tagesprofil von einem Abgabe-Tagesprofil, welches dem um die erste Zeitverschiebung verschobenen ersten Abgabe-Tagesprofil entspricht, abweicht, und wobei diese Abweichung bei der Ermittlung der weiteren, in Abhängigkeit von der zweiten Zeitverschiebung ermittelten Abgabe-Tagesprofile berücksichtigt wird.

4. Verfahren nach einem der vorangehenden Ansprüche, wobei ein Teil oder alle der weiteren Abgabe-Tagesprofile einem zeitlich um eine bestimmte Zeitverschiebung verschobenen ersten Abgabe-Tagesprofil entsprechen.

5. Verfahren nach Anspruch 3 und nach Anspruch 4, wobei zumindest die auf das zuletzt am zweiten Ort verwendete Abgabe-Tagesprofil folgenden weiteren Abgabe-Tagesprofile jeweils einem zeitlich verschobenen ersten Abgabe-Tagesprofil entsprechen und das zuletzt am zweiten Ort verwendeten Abgabe-Tagesprofil einem lediglich um einen Teilbetrag der ersten Zeitverschiebung verschobenen ersten Abgabe-Tagesprofil entspricht, und wobei der Differenzbetrag zwischen diesem Teilbetrag und dem Gesamtbetrag der ersten Zeitverschiebung bei der Ermittlung der weiteren, in Abhängigkeit von der zweiten Zeitverschiebung ermittelten Abgabe-Tagesprofile berücksichtigt wird, insbesondere indem er vorzeichenrichtig mit der zweiten Zeitverschiebung aufsummiert wird.

6. Verfahren nach einem der Ansprüche 4 bis 5, wobei die Zeitverschiebungsdifferenz zwischen jeweils zwei direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, einem Zeitbetrag entspricht, der durch ganzzahlige Teilung einer zur Ermittlung mindestens eines dieser Abgabe-Tagesprofile herangezogenen Zeitverschiebung erhalten wurde, und insbesondere, wobei dieser Zeitbetrag kleiner oder gleich 4 Stunden ist, insbesondere kleiner oder gleich 3 Stunden ist.

7. Verfahren nach Anspruch 6, wobei die Zeitverschiebungsdifferenzen zwischen allen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, identisch sind.

8. Verfahren nach einem der Ansprüche 4 bis 7, wobei eine gewünschte oder maximal zulässige Zeitverschiebungsdifferenz zwischen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, der Steuerung durch manuelle Eingabe oder über eine insbesondere drahtlose Schnittstelle mitgeteilt wird oder in der Steuerung abgespeichert ist und wobei mit der Steuerung in Abhängigkeit von der ersten oder der zweiten Zeitverschiebung und von der gewünschten oder zulässigen Zeitverschiebungsdifferenz die weiteren Abgabe-Tagesprofile ermittelt werden.

9. Verfahren nach einem der vorangehenden Ansprüche, wobei ein Teil oder alle der weiteren Abgabe-Tagesprofile einem im wesentlichen stufenlos oder an einer oder mehreren Stellen zeitlich gestauchten und/oder gedehnten ersten Abgabe-Tagesprofil entsprechen.

10. Verfahren nach Anspruch 9, wobei die Dehnung oder Stauchung intervallweise um jeweils einen bestimmten Zeitbetrag erfolgt, insbesondere jede Stunde, alle 2 Stunden, alle 4 Stunden oder alle 6 Stunden um einen bestimmten Zeitbetrag, und insbesondere, wobei der Zeitbetrag bei allen Dehnungen oder Stauchungen identisch ist oder über das Abgabe-Tagesprofil gesehen Dehnungen und/oder Stauchungen mit verschiedenen Zeitbeträgen erfolgen.

11. Verfahren nach einem der Ansprüche 9 bis 10, wobei alle gedehnten oder gestauchten Abgabe-Tagesprofile eine identische Profilform aufweisen und zeitlich zueinander verschoben sind.

12. Verfahren nach einem der Ansprüche 9 bis 11, wobei ein gewünschter oder maximal zulässiger Zeitbetrag je Dehnung bzw. Stauchung und/oder eine gewünschte oder maximal und/oder minimal zulässige Anzahl von Dehn- bzw. Stauchschritten und/oder ein gewünschtes oder maximal und/oder minimal zulässiges Intervall zwischen den Dehn- bzw. Stauchschritten der Steuerung durch manuelle Eingabe oder über eine insbesondere drahtlose Schnittstelle mitgeteilt wird oder in der Steuerung abgespeichert ist und mit der Steuerung automatisch in Abhängigkeit von der ersten Zeitverschiebung und von dem gewünschten oder zulässigen Zeitbetrag je Dehnung bzw. Stauchung und/oder von der gewünschten oder maximal und/oder minimal zulässigen Anzahl von Dehn- bzw. Stauchschritten und/oder von dem gewünschten oder maximal und/oder minimal zulässigen Intervall zwischen den Dehn- bzw. Stauchschritten die weiteren Abgabe-Tagesprofile ermittelt werden.

13. Verfahren nach einem der vorangehenden Ansprüche, wobei zumindest ein Teil der weiteren Abgabe-Tagesprofile einem zeitlich um eine bestimmte Zeitverschiebung verschobenen und zugleich zeitlich gestauchten oder zeitlich gedehnten ersten Abgabe-Tagesprofil entspricht.

14. Verfahren nach einem der vorangehenden Ansprüche, wobei ein auf das oder die weiteren Abgabe-Tagesprofile folgendes Abgabe-Tagesprofil dem um die erste Zeitverschiebung oder um die Summe aus erster und zweiter Zeitverschiebung verschobenen ersten Abgabe-Tagesprofil entspricht.

15. Verfahren nach einem der vorangehenden Ansprüche, wobei die erste und gegebenenfalls die zweite Zeitverschiebung der Steuerung durch manuelle Eingabe oder durch Datenübertragung über eine insbesondere drahtlose Schnittstelle mitgeteilt wird oder mit der Steuerung durch Auswertung, insbesondere drahtlos empfangener Daten, insbesondere über ein Satelliten-Navigationssystems empfangener Daten, ermittelt wird.

16. Verfahren nach einem der vorangehenden Ansprüche, wobei eine computergestützte Steuerung mit mindestens zwei getrennten, insbesondere gemeinsam getakteten Uhren (3, 4) verwendet wird, derart, dass mit einer ersten der Uhren (3) die zweite Ortszeit (t2) oder die dritte Ortszeit verwaltet wird, und insbesondere über ein Display (1) an der Dosiervorrichtung angezeigt wird, und mit einer zweiten der Uhren (4) die Steuerung der Dosiervorrichtung gemäss einem Abgabe-Tagesprofil erfolgt, wobei die zweite der Uhren (4) zur Steuerung der Dosiervorrichtung gemäss einem weiteren Abgabe-Tagesprofil mit der Steuerung automatisch derartig eingestellt wird, dass sie einen Zeitversatz gegenüber der ersten Ortszeit (t1) aufweist, der kleiner als die erste Zeitverschiebung oder die Summe aus der ersten und der zweiten Zeitverschiebung ist.

17. Verfahren nach Anspruch 4 und nach Anspruch 16, wobei die zweite der Uhren (4) über die Steuerung automatisch derartig eingestellt wird, dass sie jeweils einen Zeitversatz gegenüber der ersten Ortszeit (t1) aufweist, welcher der Zeitverschiebung eines gerade zur Steuerung der Dosiervorrichtung verwendeten weiteren Abgabe-Tagesprofil gegenüber dem ersten Abgabe-Tagesprofil entspricht.

18. Verfahren nach einem der vorangehenden Ansprüche, wobei ein Grad der Umstellung des Betriebs der Dosiervorrichtung auf die zweite Ortszeit (t2) oder auf die dritte Ortszeit an der Dosiervorrichtung graphisch dargestellt wird, insbesondere derart, das Abstände zwischen graphischen Elementen und/oder die Erstreckung von graphischen Elementen der Darstellung von Zeitdifferenzen dienen.

19. Verfahren nach Anspruch 18, wobei das Verhältnis der ersten Ortszeit (t1), der zweiten Ortszeit (t2) und/oder der dritten Ortszeit sowie der aktuelle Grad der Umstellung des Betriebs der Dosiervorrichtung auf die zweite Ortszeit (t2) oder die dritte Ortszeit, insbesondere durch Piktogramme, deren Abstände (a, b, B) zueinander der Darstellung der jeweiligen Zeitdifferenzen dienen und insbesondere proportional zu den jeweiligen Zeitdifferenzen sind, grafisch dargestellt wird.

20. Verfahren nach Anspruch 17 und nach einem der Ansprüche 18 bis 19, wobei der aktuelle Grad der Umstellung des Betriebs der Dosiervorrichtung dadurch dargestellt wird, dass die aktuelle Zeit der zweiten Uhr (4) angezeigt und/oder im Verhältnis zur zweiten Ortszeit (t2) oder dritten Ortszeit und insbesondere zusätzlich zur ersten Ortszeit (t1) dargestellt wird.

21. Verfahren nach einem der vorangehenden Ansprüche, wobei die zweite Ortszeit (t2) und/oder die dritte Ortszeit und insbesondere zusätzlich die erste Ortszeit (t1) an der Dosiervorrichtung als analoge oder digitale Uhrenanzeige angezeigt werden.

22. Verfahren nach einem der vorangehenden Ansprüche, wobei mit der Dosiervorrichtung zusätzlich Boli berechnet werden, in Abhängigkeit vom aktuellen Grad der Umstellung des Betriebs der Dosiervorrichtung auf die zweite Ortszeit (t2) oder die dritte Ortszeit.

23. Verfahren nach Anspruch 22, wobei die Boli derartig berechnet werden, dass in der Umstellungsphase vorübergehend eine grössere Abweichung vom sonst üblichen Zielwert oder Zielwertkorridor eines Parameters zugelassen wird.

24. Verfahren nach einem der vorangehenden Ansprüche, wobei Steuerungsdaten der Dosiervorrichtung als Steuerungs-Historie mit Referenz auf die erste Ortszeit (t1) abgespeichert werden und insbesondere, wobei zumindest bei der Speicherung der Daten von therapierelevanten Informationen, insbesondere von Bolusgaben, zusätzlich die zweite Ortszeit (t2) oder die dritte Ortszeit abgespeichert wird.

25. Anwendung des Verfahrens zum Betrieb einer computergesteuerten Insulinpumpe.

26. Dosiervorrichtung für flüssige Medikamente, insbesondere zur Durchführung des Verfahrens nach einem der Ansprüche 1 bis 24, umfassend eine Fördereinrichtung zur Förderung des flüssigen Medikaments sowie eine computergestützte Steuerung zur automatischen Steuerung der Fördereinrichtung gemäss einem ersten, in der Steuerung abgespeicherten Abgabe-Tagesprofil, wobei die Steuerung derartig ausgestaltet ist, dass mit ihr bei einer insbesondere reisebedingten ersten Zeitverschiebung mindestens ein weiteres Abgabe-Tagesprofil, welches von dem ersten Abgabe-Tagesprofil und dem um die erste Zeitverschiebung verschobenen ersten Abgabe-Tagesprofil verschieden ist, in Abhängigkeit von der ersten Zeitverschiebung ermittelbar ist und die Fördereinrichtung automatisch gemäss diesem mindestens einen weiteren Abgabe-Tagesprofil steuerbar ist.

27. Dosiervorrichtung nach Anspruch 26, wobei die Steuerung derartig ausgestaltet ist, dass mit ihr bei einer an die erste Zeitverschiebung anschliessenden zweiten Zeitverschiebung mindestens ein weiteres Abgabe-Tagesprofil, welches von dem zuletzt verwendeten Abgabe-Tagesprofil und dem um die vorzeichenrichtige Summe aus der ersten und der zweiten Zeitverschiebung verschobenen ersten Abgabe-Tagesprofil verschieden ist, in Abhängigkeit von der zweiten Zeitverschiebung ermittelbar ist und die Fördereinrichtung automatisch gemäss diesem in Abhängigkeit von der zweiten Zeitverschiebung ermittelten mindestens einen weiteren Abgabe-Tagesprofil steuerbar ist.

28. Dosiervorrichtung nach Anspruch 27, wobei die Steuerung derartig ausgestaltet ist, dass mit ihr für den Fall, dass das zuletzt zwecks Anpassung an die erste Zeitverschiebung verwendete Abgabe-Tagesprofil von dem um die erste Zeitverschiebung verschobenen ersten Abgabe-Tagesprofil abweicht, diese Abweichung bei der Ermittlung des in Abhängigkeit von der zweiten Zeitverschiebung ermittelten mindestens einen weiteren Abgabe-Tagesprofils zusätzlich zur zweiten Zeitverschiebung berücksichtigbar ist, insbesondere automatisch berücksichtigt wird.

29. Dosiervorrichtung nach einem der Ansprüche 27 bis 28, wobei die Steuerung derartig ausgestaltet ist, dass mit ihr weitere Abgabe-Tagesprofile ermittelbar und zur automatischen Steuerung verwendbar sind, welche einem zeitlich um eine bestimmte Zeitverschiebung verschobenen ersten Abgabe-Tagesprofil entsprechen.

30. Dosiervorrichtung nach Anspruch 28 und nach Anspruch 29, wobei die Steuerung derartig ausgestaltet ist, dass eine bei Ermittlung der weiteren Abgabe-Tagesprofile in Abhängigkeit von der zweiten Zeitverschiebung vorhandene Zeitverschiebungsdifferenz zwischen der ersten Zeitverschiebung und der Zeitverschiebung des zuletzt verwendeten Abgabe-Tagesprofils automatisch bei der Ermittlung des mindestens einen weiteren Abgabe-Tagesprofils berücksichtigt wird, insbesondere indem diese Zeitverschiebungsdifferenz vorzeichenrichtig mit der zweiten Zeitverschiebung aufsummiert wird.

31. Dosiervorrichtung nach einem der Ansprüche 29 bis 30, wobei die Steuerung derartig ausgestaltet ist, dass die Zeitverschiebungsdifferenz zwischen zwei direkt aufeinander folgenden Abgabe-Tagesprofilen, welche dem ersten Abgabe-Tagesprofil bzw. einem zeitlich um eine bestimmte Zeitverschiebung verschobenen ersten Abgabe-Tagesprofil entsprechen, automatisch derartig einstellbar ist, dass diese einem Zeitbetrag entspricht, der durch ganzzahlige Teilung der zur Ermittlung eines der Abgabe-Tagesprofile herangezogenen Zeitverschiebung erhalten wurde, und insbesondere, wobei die Steuerung derartig ausgestaltet ist, dass sie diese Zeitverschiebungsdifferenz automatisch auf einen Betrag kleiner oder gleich 4 Stunden, insbesondere kleiner oder gleich 3 Stunden begrenzt.

32. Dosiervorrichtung nach Anspruch 31, wobei die Steuerung derartig ausgestaltet ist, dass mit ihr die Zeitverschiebungsdifferenzen zwischen allen direkt aufeinander folgenden Abgabe-Tagesprofilen, welche dem ersten Abgabe-Tagesprofil bzw. einem zeitlich um eine bestimmte Zeitverschiebung verschobenen ersten Abgabe-Tagesprofil entsprechen, identisch einstellbar sind, insbesondere automatisch identisch eingestellt werden können.

33. Dosiervorrichtung nach einem der Ansprüche 29 bis 32, wobei die Steuerung derartig ausgestaltet ist, dass eine gewünschte oder maximal zulässige Zeitverschiebungsdifferenz zwischen direkt aufeinander folgenden Abgabe-Tagesprofilen, welche dem ersten Abgabe-Tagesprofil oder einem zeitlich um eine bestimmte Zeitverschiebung verschobenen ersten Abgabe-Tagesprofil entsprechen, der Steuerung durch manuelle Eingabe oder über eine insbesondere drahtlose Schnittstelle mitgeteilt werden kann oder in der Steuerung abgespeichert ist und mit der Steuerung in Abhängigkeit von der ersten oder der zweiten Zeitverschiebung und von der gewünschten oder zulässigen Zeitverschiebungsdifferenz die weiteren Abgabe-Tagesprofile ermittelbar sind.

34. Dosiervorrichtung nach einem der Ansprüche 26 bis 33, wobei die Steuerung derartig ausgestaltet ist, dass mit ihr weitere Abgabe-Tagesprofile ermittelbar und zur automatischen Steuerung verwendbar sind, welche einem im wesentlichen stufenlos oder an einer oder mehreren Stellen zeitlich gestauchten und/oder gedehnten ersten Abgabe-Tagesprofil entsprechen.

35. Dosiervorrichtung nach Anspruch 34, wobei die Steuerung derartig ausgestaltet ist, dass die Dehnung oder Stauchung intervallweise um jeweils einen bestimmten Zeitbetrag erfolgt oder erfolgen kann, insbesondere jede Stunde, alle 2 Stunden, alle 4 Stunden oder alle 6 Stunden, und insbesondere, dass die Steuerung derartig ausgestaltet ist, dass der Zeitbetrag bei allen Dehnungen oder Stauchungen identisch einstellbar oder eingestellt ist oder über das Abgabe-Tagesprofil gesehen verschiedene Zeitbeträge der Dehnungen bzw. Stauchungen einstellbar oder eingestellt sind.

36. Dosiervorrichtungen nach einem der Ansprüche 34 bis 35, wobei die Steuerung derartig ausgestaltet ist, dass mit ihr mehrere aufeinander folgende zeitlich gedehnte und/oder gestauchte weitere Abgabe-Tagesprofile ermittelbar und zur automatischen Steuerung verwendbar sind, welche alle eine identische Profilform aufweisen und zeitlich zueinander verschoben sind.

37. Dosiervorrichtung nach einem der Ansprüche 34 bis 36, wobei die Steuerung derartig ausgestaltet ist, dass ein gewünschter oder maximal zulässiger Zeitbetrag je Dehnung bzw. Stauchung, eine gewünschte oder maximal und/oder minimal zulässige Anzahl von Dehn- und/oder Stauchschritten und/oder ein gewünschtes oder maximal und/oder minimal zulässiges Intervall zwischen den Dehn- bzw. Stauchschritten der Steuerung durch manuelle Eingabe oder über eine insbesondere drahtlose Schnittstelle mitgeteilt werden kann oder in der Steuerung abgespeichert sind und mit der Steuerung automatisch in Abhängigkeit von der ersten Zeitverschiebung und von dem gewünschten oder zulässigen Zeitbetrag je Dehnung bzw. Stauchung und/oder von der gewünschten oder maximal und/oder minimal zulässigen Anzahl von Dehn- bzw. Stauchschritten und/oder von dem gewünschten oder maximal und/oder minimal zulässigen Intervall zwischen den Dehn- bzw. Stauchschritten die weiteren Abgabe-Tagesprofile ermittelbar sind.

38. Dosiervorrichtung nach einem der Ansprüche 26 bis 37, wobei die Steuerung derartig ausgestaltet ist, dass mit ihr weitere Abgabe-Tagesprofile ermittelbar und zur automatischen Steuerung einsetzbar sind, welche einem zeitlich um eine bestimmte Zeitverschiebung verschobenen und zugleich zeitlich gestauchten oder zeitlich gedehnten ersten Abgabe-Tagesprofil entsprechen.

39. Dosiervorrichtung nach einem der Ansprüche 26 bis 38, wobei die Steuerung derartig ausgestaltet ist, dass ein auf das oder die mit ihr ermittelten weiteren Abgabe-Tagesprofile folgendes Abgabe-Tagesprofil dem um die erste Zeitverschiebung oder um die Summe aus der ersten und der zweiten Zeitverschiebung verschobenen ersten Abgabe-Tagesprofil entspricht.

40. Dosiervorrichtung nach einem der Ansprüche 26 bis 39, wobei die Steuerung derartig ausgestaltet ist, dass die jeweilige Zeitverschiebung der Steuerung durch manuelle Eingabe oder durch Datenübertragung über eine insbesondere drahtlose Schnittstelle mitgeteilt werden kann oder mit der Steuerung durch Auswertung insbesondere drahtlos empfangener Daten, insbesondere mittels eines Satelliten-Navigationssystems, ermittelbar ist.

41. Dosiervorrichtung nach einem der Ansprüche 26 bis 40, wobei die Steuerung mit mindestens zwei getrennten, insbesondere gemeinsam getakteten Uhren (3, 4) ausgerüstet ist und derartig ausgebildet ist, dass sie mit einer ersten der Uhren (3) eine um die erste oder zweite Zeitverschiebung verschobene Zeit (t2) verwaltet und insbesondere diese Zeit (t2) über ein Display (1) an der Dosiervorrichtung anzeigt, und mit einer zweiten der Uhren (4) die Dosiervorrichtung gemäss einem Abgabe-Tagesprofil steuert, wobei die zweite der Uhren (4) zur Steuerung der Dosiervorrichtung gemäss einem weiteren Abgabe-Tagesprofil mit der Steuerung automatisch derartig einstellbar ist, dass sie einen Zeitversatz gegenüber einer Basiszeit (t1) vor der ersten Zeitverschiebung aufweist, welcher kleiner als die erste Zeitverschiebung oder die Summe aus erster und zweiter Zeitverschiebung ist.

42. Dosiervorrichtung nach Anspruch 29 und nach Anspruch 41, wobei die zweite der Uhren (4) über die Steuerung automatisch derartig einstellbar ist, dass sie jeweils einen Zeitversatz gegenüber der Basiszeit (t1) aufweist, welcher der Zeitverschiebung eines gerade zur Steuerung der Dosiervorrichtung verwendeten weiteren Abgabe-Tagesprofil gegenüber dem ersten Abgabe-Tagesprofil entspricht.

43. Dosiervorrichtung nach einem der Ansprüche 26 bis 42, wobei die Dosiervorrichtung Anzeigemittel (1) aufweist, mit denen ein Grad der Anpassung des Betriebs der Dosiervorrichtung an die erste oder zweite Zeitverschiebung an der Dosiervorrichtung graphisch anzeigbar ist, insbesondere derart, das Abstände (a, b, B) zwischen graphischen Elementen und/oder die Erstreckung von graphischen Elementen der Darstellung von Zeitdifferenzen dienen.

44. Dosiervorrichtung nach Anspruch 43, wobei das Verhältnis der Basiszeit (t1), einer um die erste Zeitverschiebung oder die Summe aus erster und zweiter Zeitverschiebung gegenüber der Basiszeit verschobenen Zeit (t2) sowie der aktuelle Grad der Anpassung des Betriebs der Dosiervorrichtung auf die erste oder zweite Zeitverschiebung insbesondere durch Piktogramme, deren Abstände (a, b, B) zueinander der Darstellung der jewieligen Zeitdifferenzen dienen und insbesondere proportional zu den jeweiligen Zeitdifferenzen sind, grafisch darstellbar ist.

45. Dosiervorrichtung nach Anspruch 42 und nach einem der Ansprüche 43 bis 44, wobei der aktuelle Grad der Anpassung an die Zeitverschiebung durch Anzeigen und/oder Darstellen der aktuellen Zeit (tP) der zweiten Uhr (4) im Verhältnis einer um die erste Zeitverschiebung oder die Summe aus erster und zweiter Zeitverschiebung gegenüber der Basiszeit (t1) verschobenen Zeit (t2) und insbesondere zusätzlich zur Basiszeit (t1) darstellbar ist.

46. Dosiervorrichtung nach einem der Ansprüche 26 bis 45, wobei die um die erste Zeitverschiebung oder die Summe aus erster und zweiter Zeitverschiebung gegenüber der Basiszeit (t1) verschobene Zeit (t2) und insbesondere zusätzlich die Basiszeit (t1) an der Dosiervorrichtung als analoge oder digitale Uhrenanzeige anzeigbar ist.

47. Dosiervorrichtung nach einem der Ansprüche 26 bis 46, wobei die Steuerung ausgestaltet ist zur zusätzlichen Berechnung von Boli, in Abhängigkeit vom aktuellen Grad der Anpassung des Betriebs der Dosiervorrichtung an die erste oder zweite Zeitverschiebung.

48. Dosiervorrichtung nach Anspruch 47, wobei die Boli derartig berechnet werden oder berechenbar sind, dass in einer Umstellungsphase vorübergehend eine grössere Abweichung vom sonst üblichen Zielwert oder Zielwertkorridor eines Parameters zugelassen wird.

49. Dosiervorrichtung nach einem der Ansprüche 26 bis 48, wobei die Steuerung ausgestaltet ist zur Aufzeichnung der Steuerungsdaten der Dosiervorrichtung als Steuerungs-Historie mit Referenz auf die Basiszeit (t1), und insbesondere, wobei die Steuerung ausgestaltet ist zur zusätzlichen Aufzeichnung einer um die erste Zeitverschiebung oder die Summe aus erster und zweiter Zeitverschiebung gegenüber der Basiszeit (t1) verschobenen Zeit (t2), zumindest bei der Aufzeichnung der Daten von therapierelevanten Informationen, insbesondere von Bolusgaben.

50. Verwendung der Dosiervorrichtung nach einem der Ansprüche 26 bis 49 zur dosierten Förderung von Insulin.
